# EUROPEAN PATENT APPLICATION

(11) **EP 3 845 639 A1**
(43) Date of publication of application: **07.07.2021**
(21) Application number: 19856074.0
(22) Date of filing: 26.08.2019
(51) Int. Cl.: C12N 5/10, C12N 5/0784, C12N 5/0786, C12N 7/00, C12N 7/04, C12N 15/12, C12Q 1/04, G01N 33/15, G01N 33/50, A61K 39/12, A61P 31/12

(54) **METHOD FOR EVALUATING ANTI-INFECTIVE DRUGS, VACCINES, ETC. USING IMMORTALIZED MONOCYTIC CELLS AND INDUCED CELLS**

(30) Priority: 27.08.2018 JP 2018158512
(71) Applicant: Mican Technologies Inc., Kyoto-shi, Kyoto 615-8245 (JP); Kumamoto University, Kumamoto-shi Kumamoto 860-8555 (JP); Osaka University, Suita-shi, Osaka 565-0871 (JP)
(72) Inventor: MIYAZAKI, Kazuo, Kyoto 615-8245 (JP); YAMANAKA, Atsushi, Osaka 565-0871 (JP); OKADA, Minoru, Kyoto 615-8245 (JP); SENJYU, Satoru, Kumamoto 860-8555 (JP)
(74) Representative: J A Kemp LLP
(86) International application number: PCT/JP2019/033378
(87) International publication number: WO 2020/045368

(57) **Abstract**

The present invention has been made in view of a problem regarding stability, reproducibility, economy, and easiness of operation in studies for a monocyte- or dendritic cell-mediated infectious microorganism, and is directed to provide a method for maintenance culturing a monocyte- or dendritic cell-mediated infectious microorganism utilizing a monocyte having a proliferative capacity. The present invention is based on the finding that a dengue virus efficiently infects a proliferable human monocytic cell obtained by introducing a gene into a CD14-positive cell and a cell having a phagocytic capacity obtained by inducing the monocytic cell to differentiate (e.g., dendritic cell) and proliferates therein. Thus, provided is a novel method for evaluating a pharmaceutical such as a compound or a vaccine for treating an infection with a monocyte- or dendritic cell-mediated infectious microorganism.

## Description

### [Technical field]

The present invention relates to a method for in vitro culturing a monocyte- or dendritic cell-mediated infectious microorganism or a method for evaluating a therapeutic agent against an infection with the microorganism.

### [Background of the invention]

In infection studies, when target pathogenic microbes proliferate via monocytes or dendritic cells, studies have been conducted using previously established cell lines. For example, a dengue virus is a virus causing an infection in human and has been studied for many years. The dengue virus has been empirically studied using cell lines such as a K562 cell line derived from human chronic myelocytic leukemia, a Vero cell line derived from an African green monkey kidney epithelial cell, and a BHK cell line derived from Syrian hamster kidney. Although these cell lines are advantageously easily evaluated, there is a problem that infection or proliferation in a human living body is not necessarily reflected.

Use of human living body-derived monocytes or dendritic cells allow analysis of a mechanism or an antigen closer to in vivo, therefore, a novel pharmaceuticals or vaccine is expected to be developed therewith. In theory, infections are capable of being evaluated using human living body-derived leukocyte-like cells, and have been evaluated using cells isolated from tissues removed from individuals. For example, it has been reported that hematopoietic stem cells derived from cord blood, bone marrow, or peripheral blood can be differentiated into blood cells and thus monocytes and induced cells in vitro. Further, a method for preparing monocyte and dendritic cells, which are more differentiated, by inducing differentiation from pluripotent stem cells such as ES cells or iPS cells has also been reported (Patent Document 1 and Non-Patent Documents 1 to 9). However, the monocytes or the dendritic cells are difficult to continuously proliferate ex vivo. Therefore, when using the cells derived from the removed tissue, extraction and isolation from the individual are needed every time the cells are prepared. This is problematic due to complicated operation and high costs. Further, these methods require a period of time for inducing differentiation and provide only a small amount of monocytic cells and a dendritic-like cells differentiated therefrom, and, therefore, there is a problem that it is difficult to continuously provide an amount necessary for study. Moreover, there is a problem that reproducibility is difficult to evaluate since individuals from which the cells derived or conditions for preparing the cells may differ each time.

As such, there has been no cell which allows monocyte- or dendritic cell-mediated infectious microorganisms to be reproducibly evaluated while satisfactorily reflecting an infection or proliferation state in a human living body.

Due to such an experimental limitation, although it has been found that the monocyte- or dendritic cell-mediated infectious microorganisms are mediated by monocytes or dendritic cells, the whole picture of conditions suited for proliferating a virus has not been revealed. Therefore, also in experiments using cells removed from individuals, there is a problem that studies do not always conducted under conditions suited for studies of an object of research, i.e., the monocyte- or dendritic cell-mediated infectious microorganisms.

Meanwhile, since antigen-presenting cells need to be prepared in a large amount when a cell therapy is administered in an immunotherapy for, such as a cancer, studies on the antigen-presenting cells such as proliferable monocytes, macrophages, or dendritic cells have been conducted. It has been reported that a gene is introduced into myeloid-based cells induced to be differentiated from iPS cells (iPS-MC), or peripheral blood-derived monocytes to thereby produce a myeloid-based cell line having a self-proliferative capacity (iPS-ML) and a monocytic cell line (CD14-ML) (Patent Documents 2 and 3).

Moreover, monocyte-derived dendritic cells have limitations as a dengue virus infection model due to its quality, proliferation, and a donor-dependent property, but dendritic cells derived from the above-mentioned iPS-ML (iPS-ML-DC) have been shown to be infected with the dengue virus and to activate HLA-matched T cells (Non-Patent Document 10). However, when using the iPS cells, there are many problems that residual iPS cells become cancerous, that stable and highly reliable induction of differentiation from the iPS cells into the myeloid-based cells is difficult, and that only the myeloid-based cells need to be reliably sorted from a population of induced cells. Further, although CD4+monocytes have an excellent ability as antigen-presenting cells (Non-Patent Document 11), the iPS-derived myeloid cells (iPS-ML) are known to lowly express CD4 (Non-Patent Document 12).

Moreover, viral isolation from infected patients is important operation for confirming, testing, and studying infection. The viral isolation is performed by collecting, for example, blood from the infected patients and operating cultured cells. For example, in the case of the dengue virus, serum samples within 5 to 6 days of the onset and C6/36 cells, Aedes albopictus cultured cell line, are commonly used (Non-Patent Document 13). However, there is a problem that the viral isolation does not necessarily succeed since viral concentrations in the serum samples and time periods from sampling to initiation of culturing affect an efficiency of the viral isolation. For example, in the case of the dengue virus, it is known that the viral concentrations in the serum samples are decreased as the patients recover and that the number of viable viruses is successively decreased after collecting the blood. Therefore, a success rate of the viral isolation is about 60% and this rate is further decreased at 5 or more days after the onset, during which a viral load is thought to be low (Non-Patent Document 14).

In addition, there is a need for cultured cells which allow culturing for mutating virus in development of vaccines. Especially, in development of attenuated vaccines, attenuated viral mutants obtained by subjecting heterologous cultured cells to repeated passages are used as vaccines. However, a long examination period, i.e., culture period is needed since viral mutation is extremely rare. For example, in the case of a rotavirus vaccine (RV1) which is an attenuated vaccine, it has been reported that the vaccine was capable of being attained only after human viruses are subjected to 33 passages in an established cell line of African green monkey kidney, selected by limiting dilution three times, and then subjected to 7 passages in Vero cells (Non-Patent Document 15). As such, it is commonly known that the attenuated mutants are not capable of being attained unless the viruses undergo culturing many times.

### [Citation List]

### [Patent Literature]

Patent Literature 1: WO2008/056734
Patent Literature 2: WO2012/043651
Patent Literature 3: Japanese Unexamined Patent Application, Publication No. 2017-131136

### [Non-patent Literature]

Non-Patent Literature 1: Fairchild P. J et al., Curr Biol. (2000) 10: 1515 - 1518.
Non-Patent Literature 2: Lindmark H et al., Exp Cell Res (2004) 300: 335-344.
Non-Patent Literature 3: Zhan X et al., Lancet (2004) 364: 163 - 171.
Non-Patent Literature 4: Slukvin II et al., J Immunol (2006) 176: 2924 - 2932.
Non-Patent Literature 5: Odegaard JI et al., J Leukoc Biol (2007) 81: 711 - 719.
Non-Patent Literature 6: Su Z et al., Clin Cancer Res (2008) 14: 6207 - 6217.
Non-Patent Literature 7: Tseng SY et al., Regen Med (2009) 4: 513 - 526.
Non-Patent Literature 8: Senju S et al., Stem cells (2007) 25: 2720 - 2729.
Non-Patent Literature 9: Choi KD et al., J Clin Invest (2009) 119: 2818 - 2829.
Non-Patent Literature 10: Dao Huy Manhra et al., J Gen Virol. (2018) doi: 10. 1099/jgv. 001119.
Non-Patent Literature 11: G. Szabo et al., J. Leukoc. Biol. (1990); 47 (2): 111 - 20.
Non-Patent Literature 12: Chihiro Koba et al., PLoS One. 2013; 8(6): e67567.
Non-Patent Literature 13: Kameoka, Modern media, vol. 61, No. 9, 2015 [Laboratory test update] 265.
Non-Patent Literature 14: Richiard G. Jarman et al., Am J Trop Med Hyg. 2011; 84(2): 218-223.
Non-Patent Literature 15: National Institute of Infectious Diseases. Japan. Rotavirus vaccine fact sheet; September 28, 2012.

### [Summary of the invention]

That is, a problem to be solved by the present invention is to find a method which is more stable, more reproducible, more economical, and easier in operation, in a study for a monocyte- or dendritic cell-mediated infectious microorganism. Further, an object of the present invention is to provide a method in which a mutant of a monocyte- or dendritic cell-mediated infectious microorganism can be obtained in a short culturing period.

The present inventors have been conducted studies to infect monocyte and dendritic cells prepared and produced by various methods with a dengue virus in order to seek a method for providing a monocyte and a dendritic cell suited for an infection study. As a result, it has been found that a dengue virus efficiently infects a proliferable human monocytic cell obtained by introducing a gene into a CD14-positive cell derived from human cord blood, an ES cell, or a bone marrow cell or peripheral blood, and a cell having a phagocytic capacity obtained by inducing the monocytic cell to differentiate (e.g., dendritic cell) and proliferates therein. Thus, the present invention has been completed. In particular, the virus which has infected these cells rapidly proliferates and produces a larger number of progeny viruses and virus-like particles. Thus, the present inventors have provided a novel method for research and development, for example, a method for evaluating a pharmaceutical such as a compound or a vaccine for treating an infection with a monocyte- or dendritic cell-mediated infectious microorganism. Further, the present inventors have succeeded in finding that a monocyte- or dendritic cell-mediated infectious microorganism can be proliferated to be used for producing a vaccine, and constructing, developing, and producing a mutant.

Further, the present invention solves the problems with use of an iPS cell by, in particular, using an immortalized monocytic cell. That is, a population which stably contains only a monocytic cell can be easily obtained by employing a method for immortalizing a monocytic cell. Moreover, a cell obtained by immortalizing a monocytic cell has a higher antigen-presenting capability than an iPS-derived dendritic cell due to expression of CD4, and, therefore, the cell is thought to be suitable for a vaccine study. Consequently, the present invention provides an infection model of a monocyte- or dendritic cell-mediated infectious microorganism which is excellent in CD4 expression.

Further, the present invention solves the problem regarding viral isolation which has been difficult to solve with a traditional cultured cell due to influence by such as a viral concentration in a sample. According to the finding by the present inventors, use of a proliferable human monocytic cell obtained by immortalizing a CD14-positive cell derived from an iPS cell, human cord blood, an ES cell, or a bone marrow cell or peripheral blood and a cell having a phagocytic capacity obtained by inducing the monocytic cell to differentiate (e.g., dendritic cell) allowed a virus to be sufficiently proliferated in the cell even in a small viral load. As a result, the present invention has succeeded in providing an isolation and evaluation system which improves a success rate of viral isolation. Therefore, in one aspect, the present invention provides an excellent cell for viral isolation.

Further, the present invention solves the problem that it takes a long time to obtain a virus mutant which is an attenuated virus. That is, according to the present invention, even when a proliferable human monocytic cell obtained by immortalizing a CD14-positive cell derived from an iPS cell, human cord blood, an ES cell, or a bone marrow cell or peripheral blood and a cell having a phagocytic capacity obtained by inducing the monocytic cell to differentiate (e.g., dendritic cell) are infected with a virus at a low concentration, a virus mutant which forms a different form of a plaque can be obtained without the necessity of culturing for a long period of time. As a result, the cell is thought to be suitable for a vaccine study since the virus mutant can be obtained in a short period of time. Therefore, the present invention provides a cell which is excellent in producing a mutant for studying a virus and a vaccine.

Specifically, the present invention relates to the following inventions.
(1) A method for maintenance culturing a monocyte- or dendritic cell-mediated infectious microorganism, the method including:
   infecting a monocyte into which a cMYC gene, and at least one gene selected from a BMI1 gene, an EZH2 gene, an MDM2 gene, an MDM4 gene, an HIF1A gene, a BCL2 gene, and an LYL1 gene have been introduced or a dendritic cell which has been induced to be differentiated from the monocyte with the infectious microorganism; and
   culturing the infected cell.
(2) A method for maintenance culturing a monocyte- or dendritic cell-mediated infectious microorganism, the method including:
   introducing a cMYC gene, and at least one gene selected from a BMI1 gene, an EZH2 gene, an MDM2 gene, an MDM4 gene, an HIF1A gene, a BCL2 gene, and an LYL1 gene into a monocyte;
   optionally inducing the monocyte to differentiate into a dendritic cell; infecting the resultant monocyte or dendritic cell with the infectious microorganism; and
   culturing the infected cell.
(3) A method for identifying a therapeutic agent against an infection with a monocyte- or dendritic cell-mediated infectious microorganism, the method including:
   infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism in the presence of a test drug;
   measuring an infection level with the infectious microorganism in the monocyte or the dendritic cell; and
   determining whether the test drug has a therapeutic effect against the infection based on the measured infection with the infectious microorganism,
   in which the test drug is determined to have the therapeutic effect against the infection if a level of the measured infection with the infectious microorganism is lower than a control infection level which is a level when infecting the cell with the infectious microorganism in the absence of the test drug.
(4) A method for producing a mutant of a monocyte- or dendritic cell-mediated infectious microorganism, the method including:
   infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism;
   culturing the infected monocyte or dendritic cell to thereby proliferate the infectious microorganism; and
   isolating a mutant from the proliferated infectious microorganism.
(5) A method for producing a vaccine against a monocyte- or dendritic cell-mediated infectious microorganism, the method including:
   infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism;
   culturing the infected monocyte or dendritic cell to thereby proliferate the infectious microorganism; and
   collecting the proliferated infectious microorganism.
(6) The method according to (6), further including inactivating the collected infectious microorganism.
(7) A method for separating the infectious microorganism from blood derived from a patient infected with a monocyte- or dendritic cell-mediated infectious microorganism, the method including:
   bringing a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte into contact with the blood derived from a patient;
   culturing the infected cell; and
   detecting and/or identifying a microorganism in the cultured cell to thereby specify the infectious microorganism.
(8) The method according to any one of (1) to (7), in which the monocyte is a cell obtained by inducing a human pluripotent stem cell or a human hematopoietic stem cell to differentiate, or a cell obtained by isolating from human peripheral blood.
(9) The method according to (8), in which the human pluripotent stem cell is an iPS cell.
(10) The method according to any one of (1) to (7), further including preparing the monocyte by inducing a human pluripotent stem cell or a human hematopoietic stem cell to differentiate, or preparing the monocyte by isolating from human peripheral blood.
(11) The method according to (10), in which the human pluripotent stem cell is an iPS cell.
(12) The method according to any one of (1) to (11), in which the infectious microorganism is a dengue virus.
(13) The method according to any one of (1) to (12), in which the at least one gene selected from a BMI1 gene, an EZH2 gene, an MDM2 gene, an MDM4 gene, an HIF1A gene, a BCL2 gene, and an LYL1 gene is a BMI1 gene and a BCL2 gene or an LYL1 gene.

### [Effect of the invention]

The present invention allows study and evaluation of a microorganism causing a monocyte- or dendritic cell-mediated infection. For example, the present invention can be used to culture and evaluate even a disease which is difficult to study due to difficulty of culturing and evaluation in a blood cell material derived from human donate blood, making it possible to study the infection and develop an anti-infective agent and a vaccine thereagainst. Moreover, a method of the present invention allows drug screening evaluation with a reduced error in development of a therapeutic agent against a blood-related disease.

### [Brief description of the drawings]

[FIG. 1]

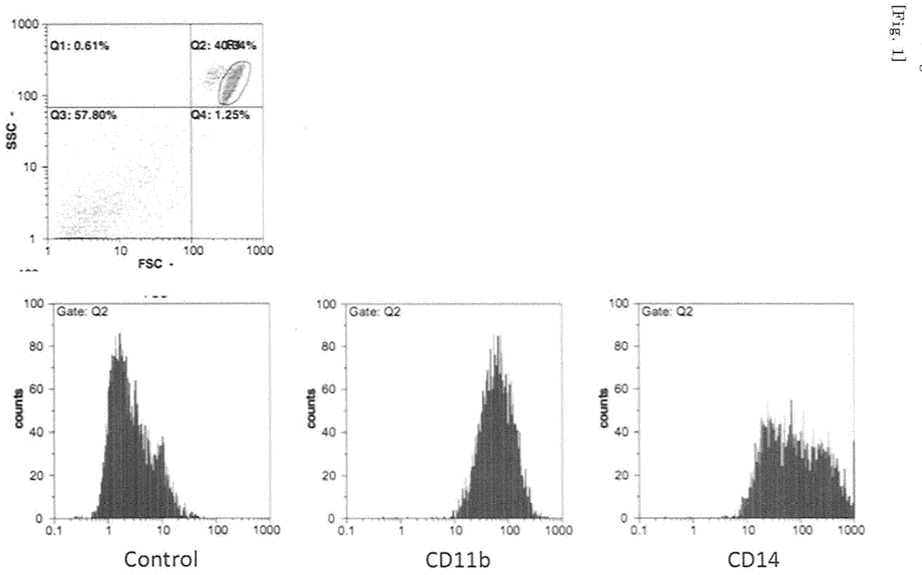

   FIG. 1 is a plot illustrating an analysis result of expression of surface markers CD11 and CD14 in the produced immortalized human monocytic cells with a flow cytometer. Antibodies used: an anti-CD11 antibody and an anti-CD14 antibody which are reactive with FITC.
[FIG. 2]

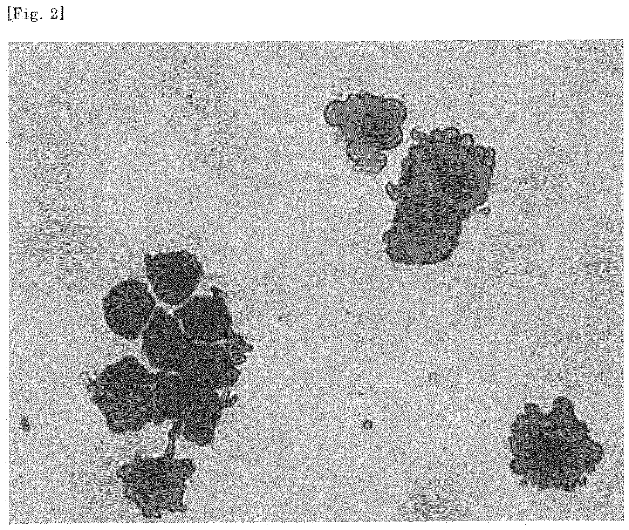

   FIG. 2 is a photograph of May-Giemsa-stained dendritic cell-like cells which were induced by stimulating immortalized human monocytic cells with IL4.
[FIG. 3]

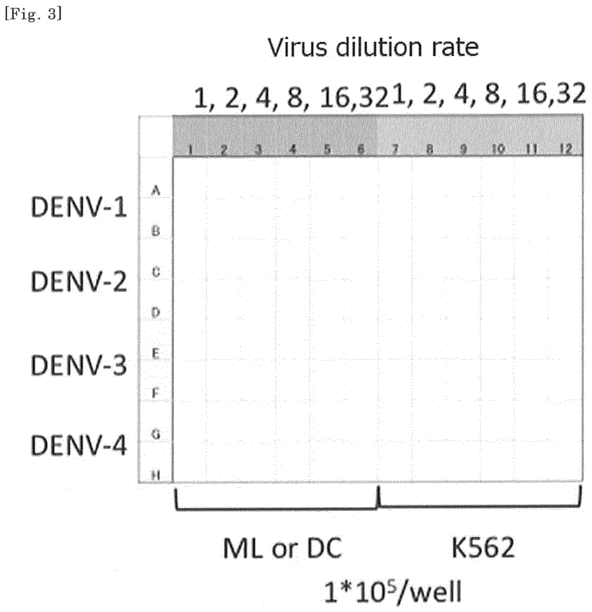

   FIG. 3 is a plate design in a dengue virus infection experiment. Human monocytic cells or dendritic cells to be evaluated were placed into a left half of a 96-well plate and K562 cells serving as a control cell were seeded into a right half. Viral solutions at different dilution rates were added into the wells at each row (1 to 12). Different kinds of dengue viruses (DENV-1: dengue virus type 1, DENV-2: dengue virus type 2, DENV-3: dengue virus type 3, and DENV-4: dengue virus type 4) for each column (A to H) were added.
[FIG. 4]

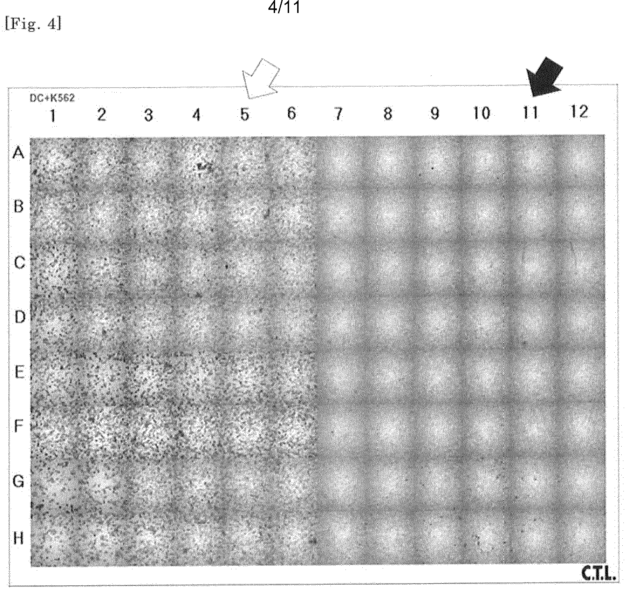

   FIG. 4 is a photograph illustrating comparison results of human dendritic-like cells prepared in Example 1 with K562 cells serving as a positive control using the plate design in FIG. 3. After a dengue virus infection experiment, virus infection was visualized by staining.
[FIG. 5]

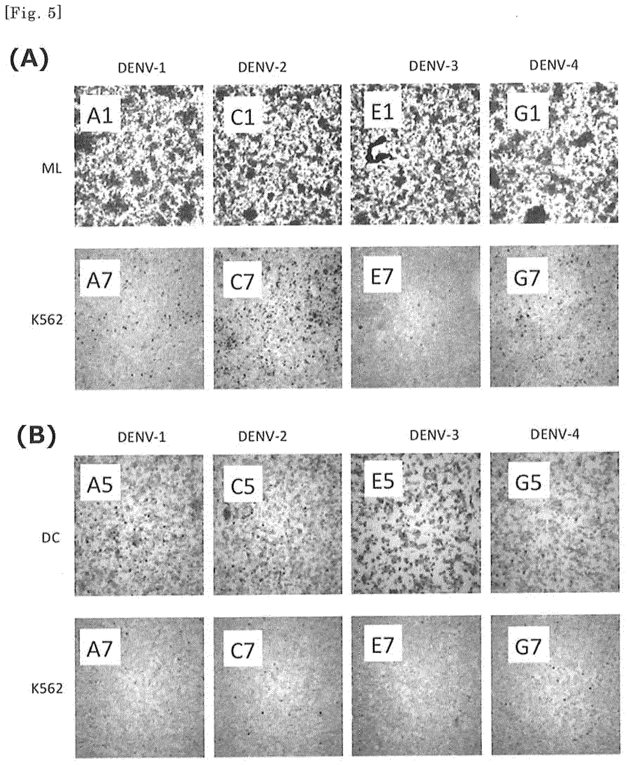

   FIG. 5 is enlarged photographs of a portion of the plate in FIG. 4. (A) Photographs of results using monocyte-like cells (ML: monocyte-like cell, K562: K562 cell line). From left, enlarged photographs of A1, C1, E1, and G1 wells (top) in FIG. 4 and A7, C7, E7, and G7 wells (bottom) in FIG. 4. (B) Photographs of evaluation results using dendritic-like cells (DC: monocyte-like cell, K562: K562 cell line). From left, enlarged photographs of A5, C5, E5, and G5 wells (top) in FIG. 4 and A7, C7, E7, and G7 wells (bottom) in FIG. 4. It is noted that A1 refers to Row A Column 1 of the plate in FIG. 4.
[FIG. 6]

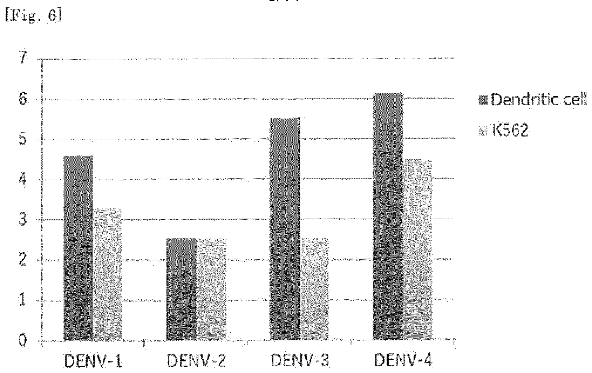

   FIG. 6 is a graph illustrating measurement results of viral release amounts from monocyte-like cells (A) and dendritic cell-like cells (B). A vertical axis represents a viral release amount into culture supernatant (log10 plaque-forming unit (PFU) / ml). A horizontal axis represents dengue virus type 1 (DENV-1) (D1), type 2 (DENV-2) (D2), type 3 (DENV-3) (D3), and type 4 (DENV-4) (D4) from left.
[FIG. 7]

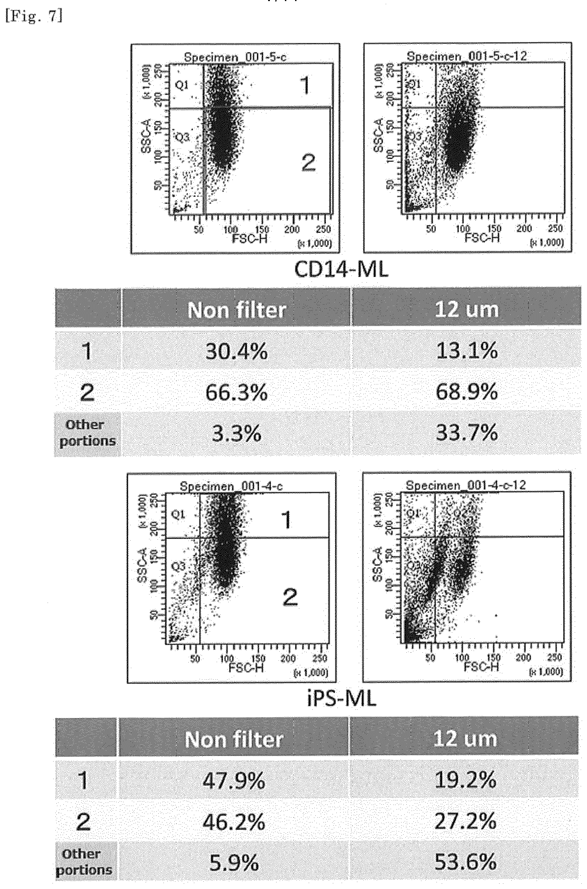

   FIG. 7 is a plot illustrating results of analysis with a flow cytometer of the produced immortalized human monocytic cells (peripheral blood-derived monocytic cells: CD14-ML, iPS cell-derived monocytic cells: iPS-ML) after being filtered, in order to measure physical purification and membrane strength in the cells. An unfiltered group which was not purified with a filter (Non filter) and a group which was purified with a mesh filter with a mesh size of 12 µm (12 µm) were measured with a flow cytometer. A vertical axis represents an area of side scattered light (SSC-A), and a horizontal axis represents a detection result of forward scattered light (FSC-H). In order to visualize the degree of purification, the plot is divided for convenience and abundance in Areas 1, 2, and the other is presented. For ensuring reproducibility in the evaluation, abundance of the number of cells in the Area 2 falls within a certain range, thereby a management is oriented.
[FIG. 8]

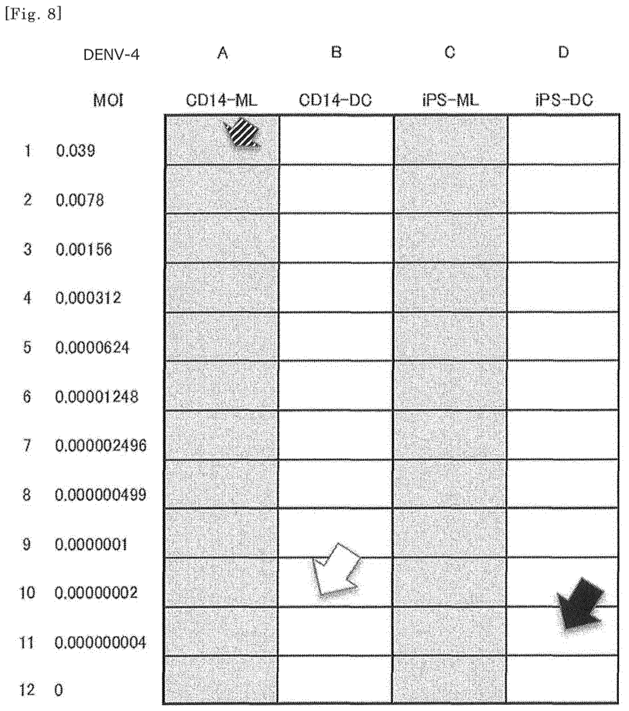

   FIG. 8 is a plate design in an infection evaluation in which a virus dilution rate is higher and a virus concentration is lower than in the dengue virus infection experiment in FIG. 3. This is based on an experiment using a dengue virus type 4 (DENV-4). To each well, were added 1 x 10⁵ cells described in A to D, and then the virus in accordance with multiplicity of infection (MOI). For example, peripheral blood-derived immortalized monocytic cells (CD14-ML) were infected with the dengue virus type 4 (DENV-4) at a rate of 0.39 (particle) per cell in A1 (striped arrow).
[FIG. 9]

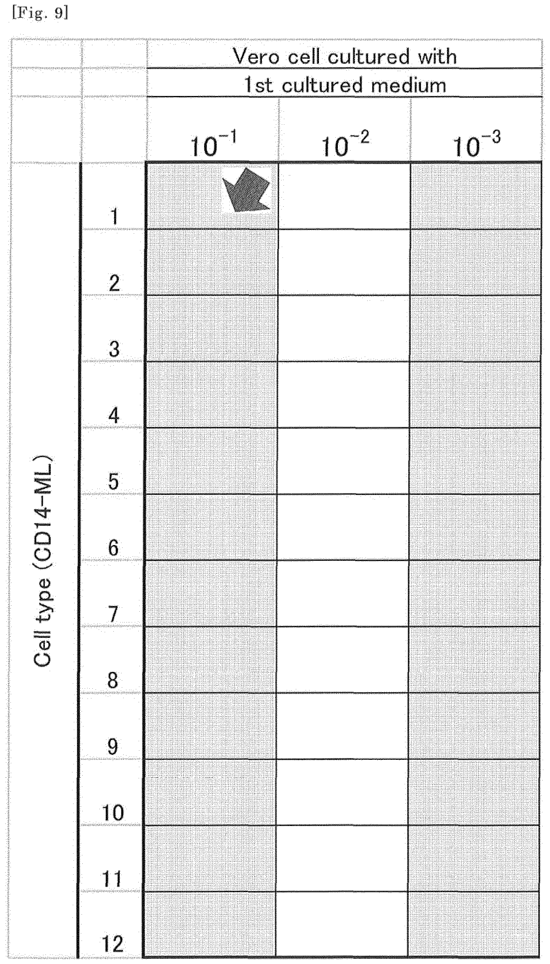

   FIG. 9 is a plate design in which a virus dilution rate is higher in the plaque dengue virus infection experiment. After the infection evaluation in FIG. 8, a supernatant was seeded into Vero cells, cultured, and then immuno-stained in order to visualize a viral load contained in the supernatant. Numerals correspond to those in FIG. 8. For example, it is a left upper well that the supernatant in A1 of FIG. 8 is diluted 10 hold and seeded thereinto.
[FIG. 10]

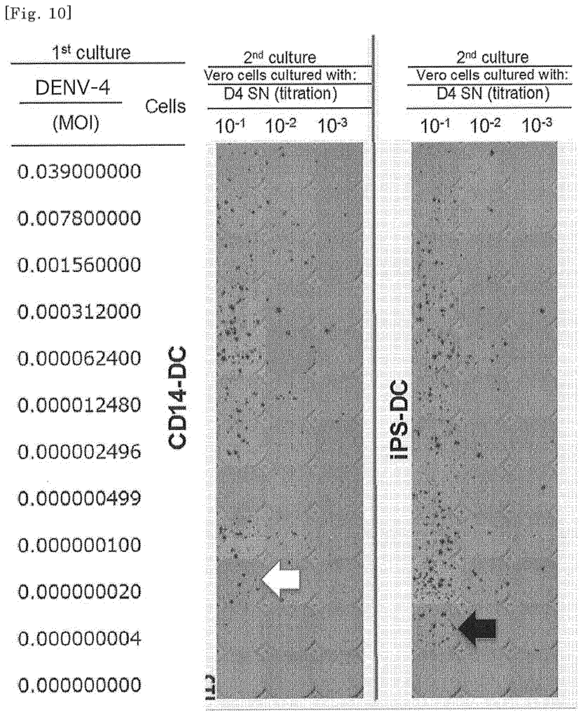

   FIG. 10 is a figure illustrating results of the plaque dengue virus infection experiment. It is shown that both peripheral blood-derived dendritic-like cells (CD14-DC: white arrow) and iPS cell-derived dendritic-like cells (iPS-DC: black arrow) proliferate and release viruses even in the presence of the viruses at an extremely low concentration.
[FIG. 11]

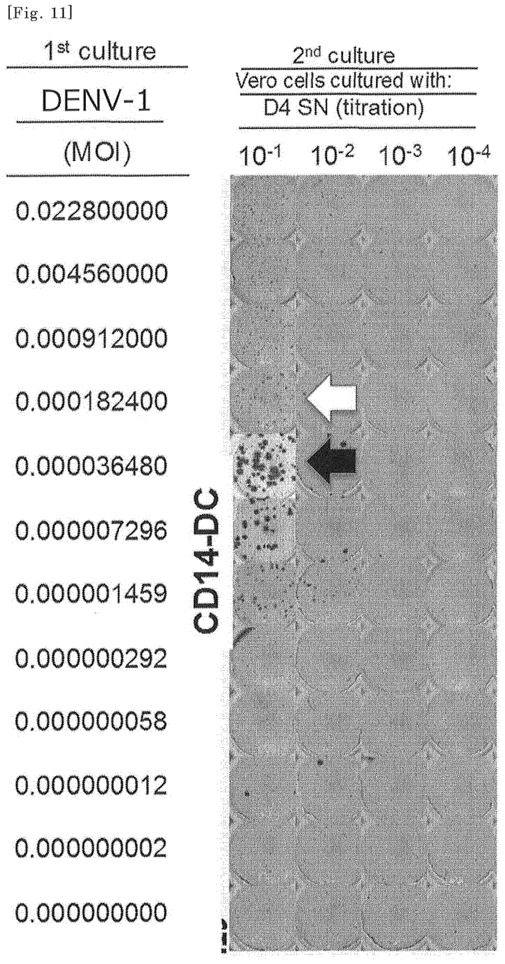

   FIG. 11 is a figure illustrating results of the plaque dengue virus infection experiment. The dengue virus type 1 infected while diluting the virus. A light spot-like plaque (white arrow) was turned into a large spot (black arrow) above a certain concentration, indicating that the virus may be mutated between them.

### [Detailed description of exemplary embodiments]

In one aspect, the present invention relates to a method for maintenance culturing a monocyte- or dendritic cell-mediated infectious microorganism, the method including infecting a monocyte into which a cMYC gene, and at least one gene selected from a BMI1 gene, an EZH2 gene, an MDM2 gene, an MDM4 gene, an HIF1A gene, a BCL2 gene, and an LYL1 gene have been introduced or a dendritic cell which has been induced to be differentiated from the monocyte with the infectious microorganism.
In another aspect, the present invention relates to a method for maintenance culturing a monocyte- or dendritic cell-mediated infectious microorganism, the method including:
introducing a cMYC gene, and at least one gene selected from a BMI1 gene, an EZH2 gene, an MDM2 gene, an MDM4 gene, an HIF1A gene, a BCL2 gene, and an LYL1 gene into a monocyte;
optionally inducing the monocyte to differentiate into a dendritic cell; and
infecting the resultant monocyte or dendritic cell with the infectious microorganism.

### <Monocyte- and dendritic cell-mediated infectious microorganism>

As used herein, the phrase "monocyte- or dendritic cell-mediated infectious microorganism" means, for example, a virus, a bacteria, and a protozoan which infects a monocyte or a dendritic cell during its survival and growth processes. Examples of the monocyte- or dendritic cell-mediated infectious microorganism include Flaviviridae virus causing the dengue fever such as a dengue virus, a Japanese encephalitis virus, a West Nile virus, a yellow fever virus, a Murray valley virus, a Saint Louis encephalitis virus, a Zika virus, and a tick-borne encephalitis virus.

### <Method for preparing monocyte>

In the present invention, a monocyte serving as a raw material can be obtained by, for example, a method of collecting from peripheral blood, a method of inducing differentiation from a pluripotent stem cell, and a method of inducing differentiation from other somatic cells such as a fibroblast. When the monocyte is collected from peripheral blood, it is known a method for isolating and preparing a cell expressing a CD14 molecule in human peripheral blood. For example, the human peripheral blood is mildly diluted with an equivalent amount of saline, phosphate buffered saline, or a Hanks buffered solution. The diluted blood is gently layered on FICOLL (registered trademark) (GE Healthcare) in a centrifuge tube and centrifuged at 500 to 1000 x g for 20 min at 15 to 30°C. A white belt-like layer between yellowish plasma and transparent FICOLL is collected as a peripheral blood mononuclear cell (PBMC) fraction composed of a lymphocyte and a monocyte. The collected peripheral blood mononuclear cell fraction may be further washed to be used, as necessary. A CD14 molecule-expressing cell can be further collected from the collected PBMC fraction to thereby obtain a monocyte. For example, a solid phase to which an anti-CD14 antibody is attached is brought into contact with PBMC to thereby allow the monocyte to bind to the solid phase, and then an unbound cell is washed to remove. Thus, the monocyte can be separated and collected. For example, it is known a method in which a magnetic bead (e.g., DYNABEADS (registered trademark) CD14 (Thermo Fisher Scientific Inc.)) is used as the solid phase. Moreover, the monocyte can also be obtained by bringing peripheral blood into direct contact with the solid phase to which the anti-CD14 antibody is attached without separating the PBMC from the peripheral blood.

When the monocyte is obtained by differentiation from a pluripotent cell, it is known a method for inducing the monocyte from the pluripotent cell. As used herein, the phrase "pluripotent stem cell" means a cell having pluripotency and a self-replication ability. As used herein, the term "pluripotency" is synonymous with multipotency, and means a state of a cell which may be differentiated into a plurality of cell lineages through differentiation. As used herein, the pluripotency includes a state in which a cell may be differentiated into all kinds of cells constituting a living body (totipotency), a state in which a cell may be differentiated into all kinds of cells except for an extraembryonic tissue (versatile differentiation), a state in which a cell may be differentiated into some of cell lineages (multipotency), and a state in which a cell may be differentiated into one kind of cell (unipotency). Therefore, as used herein, the phase "pluripotent stem cell" includes a stem cell, an embryonic stem (ES) cell, a cloned embryo-derived embryonic stem cell obtained by nuclear transplantation ("ntES cell"), a germline stem cell ("GS cell"), an embryonic germ cell ("EG cell"), and an induced pluripotent stem (iPS) cell, and a hematopoietic stem cell. As to whether a cell is the pluripotent stem cell, for example, if a test cell forms an embryoid body in an in vitro culture system or if a test cell is differentiated into a desired cell after the test cell is cultured under conditions for inducing differentiation (subjecting to a differentiation treatment), the cell may be determined as the pluripotent stem cell. A method for obtaining the pluripotent stem cell is known and, for example, it has been reported that the iPS cell is obtained by introducing three kinds or more of reprogramming genes (e.g., Oct3/4, Sox2, Klf4, c-Myc, L-Myc, Nanog, Lin28, Esrrb, Glis1, E-cadherin, shp53, UTX, and H1foo) into a somatic cell.

The pluripotent stem cell may be induced to differentiate into the monocyte by, for example, bringing a macrophage colony-stimulating factor (M-CSF) and interleukin 3 (IL-3) (see Fernadndo O. Martinez et al., Experimental Hematology (2008); 36: 1167 - 1175)), or IFN-γ or PMA (Annabelle Grolleau et al., J Immunol 1999; 162: 3491 - 3497) into contact with the pluripotent stem cell. The resultant monocyte which has been differentiated may be purified by collecting only a CD14-expressing cell by the above-mentioned method, as necessary.

A method for inducing other somatic cells such as a fibroblast to differentiate into the monocyte is described in Takahiro Suzuki et al., "Reconstruction of Monocyte Transcriptional Regulatory Network Accompanies Monocytic Functions in Human Fibroblasts." PLoS One (2012) doi: 10. 1371/journal. pone. 033474.

The resultant monocyte can be imparted with a proliferative ability while maintaining a function of the monocyte by introducing a cMYC gene, and at least one gene selected from a BMI1 gene, an EZH2 gene, an MDM2 gene, an MDM4 gene, an HIF1A gene, a BCL2 gene, and an LYL1 gene. Preferable is a combination of a cMYC gene with at least one gene selected from a BMI1 gene, an EZH2 gene, an MDM2 gene, an MDM4 gene and an HIF1A gene, or a combination of a cMYC gene with a BMI1 gene and a BCL2 gene or LYL1 gene. These genes can be introduced into the monocyte considering the description in WO2012/043651 and Japanese Unexamined Patent Application, Publication No. 2017-131136. For example, the introduction may be performed by infecting a monocyte with a lentiviral vector carrying these genes.

### <Method for preparing dendritic cell>

A dendritic cell may be induced from a monocyte by, for example, culturing the monocyte in the presence of 200 IU / ml of GM-CSF and 200 IU / ml of IL-4 according to the previous reports (Francoise Chapuis et al., Eur J Immunol. (1997) 27(2): 431 - 41.; Marc Dauer et al., J Immunol (2003) 170(8): 4069 - 4076.; Figdor CG et al., Nat Med. (2004) 10(5): 475 - 80; Helmut Jonuleit et al., Eur J Immunol. (1997) 27(12): 3135 - 42).

### <Infection and culturing of monocyte- and dendritic cell-mediated infectious microorganism>

The monocyte or the dendritic cell may be infected with the monocyte- and dendritic cell-mediated infectious microorganism by bringing a monocyte- and dendritic cell-mediated infectious microorganism-containing solution into contact with the cell. Examples of a medium used in culturing upon infection and/or after infection include an IMDM solution, an RPMI solution, an α-MEM solution, a MEM solution, and a DMEM solution. Moreover, for example, a human or bovine plasma protein fraction, fetal bovine serum or human serum, sugars such as glucose and D-mannitol, an amino acid, a nucleobase such as adenine, sodium hydrogen phosphate, α-tocopherol, linoleic acid, cholesterol, sodium selenite, human holo-transferrin, human insulin, ethanolamine, 2-mercaptoethanol, EPO, TPO, sodium hydrogen carbonate, or methylcellulose may be included as an additive. Moreover, an antibiotic such as gentamicin, ampicillin, penicillin, and streptomycin; an inorganic salt; a buffer such as HEPES, a phosphate buffer, and an acetate buffer may be added, as necessary. Specifically, an α-MEM (10% fetal bovine serum) medium supplemented with a non-essential amino acid or an RPMI 1640 medium (SIGMA) containing hypoxanthine and HEPES (SIGMA) and supplemented with 10% fetal bovine serum, sodium hydrogen carbonate, 100 unit / ml of penicillin, and 100 µg / ml of streptomycin may be used. A culturing time may be appropriately set according to kinds of cell, microorganism, and medium, but may be, for example, 24 to 96 hours, 36 to 84 hours, or 72 hours. When culturing an infected cell for obtaining a mutant, the number of times of culturing may be few, for example, 1 to 5, 1 to 4, 1 to 3, 1 to 2, or 1. Other culturing conditions may be determined according to common conditions for culturing a human cell and may be, for example, at 37°C and 5% CO₂.

### <Method for screening therapeutic agent>

In another aspect, the present invention relates to a method for identifying a therapeutic agent against an infection with a monocyte- or dendritic cell-mediated infectious microorganism, the method including:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism in the presence of a test drug;
measuring an infection level with the infectious microorganism in the monocyte or the dendritic cell; and
determining whether the test drug has a therapeutic effect against the infection based on the measured infection with the infectious microorganism.
In this method, the test drug is determined to have the therapeutic effect against the infection if a level of the measured infection with the infectious microorganism is lower than a control infection level which is a level when infecting the cell with the infectious microorganism in the absence of the test drug.

The infection level of the infectious microorganism may be measured by measuring an infectious titer of the microorganism contained in a post-culture supernatant by Cell-ELISA, immunostaining, a plaque-forming method, or a PCR method. Alternatively, the infection level may be measured by fixing a post-culture infected cell and subjecting it to the immunostaining or the Cell ELISA.

When the infection level with the monocyte or the dendritic cell with the monocyte- and dendritic cell-mediated infectious microorganism is measured by the Cell-ELISA or the immunostaining, a screening method of the present invention may include fixing an infected monocyte or dendritic cell and bringing an antibody specific for the monocyte- and dendritic cell-mediated infectious microorganism into contact with the fixed cell. For example, the Cell-ELISA method herein may include bringing an antibody specifically binding to the microorganism (primary antibody) into contact with the fixed cell, removing (washing) an unbound antibody, bringing a labeled antibody being capable of binding to the primary antibody (secondary antibody) into contact with the fixed cell which has been contacted with the primary antibody, removing (washing) an unbound antibody, measuring a labelling level (intensity, area, number, etc.) of the secondary antibody which has been bound to the fixed cell, and calculating from the measured labelling level the infection level of the cell infected with the microorganism. Alternatively, the immunostaining method herein may include bringing a labeled antibody capable of specifically binding to the microorganism into contact with the fixed cell, removing (washing) an unbound antibody, measuring a labelling level (intensity, area, number, etc.) of the antibody which has been bound to the fixed cell, and calculating from the measured labelling level the infection level of the cell infected with the microorganism. As a label used for the labelled antibody, a detectable label such as a radiolabel, an enzyme label, a fluorescent label, a bioluminescent label, a chemiluminescent label, a metal, a near-infrared light label may be used.

When the infection level of the monocyte or the dendritic cell with the monocyte- and dendritic cell-mediated infectious microorganism is measured by the plaque-forming method, the method may include mixing a microorganism-containing liquid collected from a culture supernatant or a cell homogenate of an infected monocyte or dendritic cell with a cell line which is known to be infected with the microorganism (e.g., a K562 cell line, a Vero cell line, or a BHK cell), adding the mixed cell / microorganism liquid into a medium, culturing the liquid for 12 hours to 10 days, fixing the post-culture infected cell, and bringing an antibody specific for the monocyte- and dendritic cell-mediated infectious microorganism into contact with the fixed cell. Specifically, the antibody specific for the microorganism may be brought into contact with the fixed cell by the above-mentioned Cell-ELISA method or immunostaining method.

When the infection level of the monocyte or dendritic cell with the monocyte- and dendritic cell-mediated infectious microorganism is measured by the PCR method, for example, a real-time PCR method may be performed using, as a sample, a microorganism-containing liquid collected from a culture supernatant or a cell homogenate of an infected monocyte or dendritic cell.

The "infection level" is not particularly limited as long as it is a numerical value reflecting the number of the infectious microorganism. For example, PFU, PFU / ml, an area of an infected area, microbial biomass (number), or a microbial concentration (number / ml) may be used. The "control infection level which is a level when infecting the cell with the infectious microorganism in the absence of the test drug" may be obtained simultaneous with the test drug by, as a control test, infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism in the absence of the test drug; and measuring an infection level with the infectious microorganism in the monocyte or the dendritic cell. Alternatively, the control infection level may be obtained from a previously or separately performed control test. Preferably, two levels to be compared are levels obtained from tests which are performed under the same conditions. The test drug is determined to have the therapeutic effect against the infection if a level of the measured infection in the presence of the test drug is lower than the control infection level which is measured in the absence of the test drug.

### <Method for producing mutant>

In another aspect, the present invention relates to a method for producing a mutant of a monocyte- or dendritic cell-mediated infectious microorganism, the method including:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism;
culturing the infected monocyte or dendritic cell to thereby proliferate the infectious microorganism; and
isolating a mutant from the proliferated infectious microorganism.

It is known that a mutation is introduced into a microorganism at a certain probability through proliferation. The present invention allowed the monocyte- or dendritic cell-mediated infectious microorganism to efficiently proliferate in vitro under an environment close to a living body, making it possible to simulatively introduce a mutation, which may occur in the living body, in vitro. The microorganism may be cultured for any period of time until the mutant is generated and, during culturing, the microorganism may infect a fresh cell and/or a medium may be replaced, as necessary. The mutant may be isolated by isolating and sequencing the infected microorganism. That is, the microorganism may be isolated by appropriately diluting a microorganism-containing liquid which has been cultured for any period of time, infecting a cell line which is known to be infected with the microorganism (e.g., a Vero cell line or a BHK cell) with the diluted liquid, and allowing the microorganism to form a single plaque. The single plaque may be formed in the same manner as in the above-mentioned plaque-forming method. The isolated microorganism is sequenced and the resultant sequence is compared with an original gene sequence of the microorganism upon infection. If the original gene sequence of the microorganism is different from the gene sequence of the isolated microorganism, the isolated microorganism may be identified as a mutant. The gene sequence may be analyzed using a commercially available DNA sequencer or next-generation sequencer (NGS).

The mutant is not particularly limited, but examples thereof include a mutant which is weakly pathogenic and may be used as a live vaccine and a drug-resistant mutant.

### <Method for producing vaccine>

In another aspect, the present invention relates to a method for producing a vaccine against a monocyte- or dendritic cell-mediated infectious microorganism, the method including:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism;
culturing the infected monocyte or dendritic cell to thereby proliferate the infectious microorganism; and
collecting the proliferated infectious microorganism.

The proliferated infectious microorganism may be collected from a culture supernatant or a homogenate liquid after cell homogenization. The collected microorganism may be appropriately purified, as necessary, and then used. For example, density gradient centrifugation, ultrafiltration, or affinity chromatography may be used for purification. When the microorganism is a strain of which pathogenicity has been attenuated, the resultant microorganism may be used as a living vaccine. Alternatively, the microorganism may be used as an inactivated vaccine including toxoid. When used as the inactivated vaccine, the method of the present invention may further include degrading the collected infectious microorganism and collecting an antigenic portion from the degraded product, as necessary, or, alternatively, extracting and inactivating a toxin in the infectious microorganism.

Further, the present invention encompasses a vaccine produced by the method. Further, the present invention encompasses a method for preventing an infection with the monocyte- or dendritic cell-mediated infectious microorganism including administering the vaccine to a subject who may be potentially infected with the microorganism and a method for treating an infection with the monocyte- or dendritic cell-mediated infectious microorganism including administering the vaccine to a subject who is infected with the microorganism. A vaccine formulation may be appropriately produced and administered using a method well-known in the art.

### <Method for isolating infectious microorganism>

In another aspect, the present invention relates to a method for separating the infectious microorganism from blood derived from a patient infected with a monocyte- or dendritic cell-mediated infectious microorganism, the method including:
bringing a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte into contact with the blood derived from a patient;
culturing the infected cell; and
detecting and/or identifying a microorganism in the cultured cell to thereby specify the infectious microorganism.

The infection and culturing may be performed according to the above-mentioned method. The microorganism in the cultured cell may be detected and/or identified by a method widely known in the art according to a microorganism of interest, for example, a method utilizing PCR or a method utilizing an antibody.

Throughout the description of the present invention, the monocyte or the dendritic cell is a mammalian monocyte or a mammalian dendritic cell and preferably a human monocyte or a human dendritic cell.

### [Examples]

The present invention will now be more specifically described based on Examples, but the present invention is not limited to the following Examples. All references cited throughout this application are hereby incorporated by reference in their entirety. In addition, a dengue virus, a medium and a dengue virus for an infection test used in the Examples are as follows.

### (Dengue virus)

Four serotypes of dengue virus were used for an infection evaluation. Used Strains were a Mochizuki strain (dengue type 1 virus), an NGC strain (dengue type 2 virus), an H87 strain (dengue type 3 virus), and a H241 strain (dengue type 4 virus), and all of which are prototype strains. Based on the known method, a supernatant released into a cultured solution of an infected Vero cell was used as each virus.

### (Medium for infection test)

As a medium for evaluation, an RPMI 1640 medium (SIGMA) containing hypoxanthine, HEPES (SIGMA) and supplemented with sodium hydrogen carbonate (final concentration: 0.225%), 100 unit / ml of penicillin, and a 100 µg / ml of streptomycin was used.

### (Example 1) Production of human peripheral blood-derived immortalized monocytic cell (CD14-ML) and production of dendritic cell

### (Production of human peripheral blood-derived immortalized monocytic cell)

A human peripheral blood-derived human immortalized monocytic cell was produced by reference to the previous reports (WO2012/043651 and Japanese Unexamined Patent Application, Publication No. 2017-131136). In detail, a CD14-positive fraction was taken from human peripheral blood and a gene reported as a mouse immortalized hematopoietic stem cell (Myc, Myb, Hob8, TLX1, E2A-pbx1, MLL, Ljx2, RARA, Hoxa9, Notch1, v-raf/v-myc, MYST3-NCOA2, Evil, HOXB6, HOXB4, β-catenin, Id1) or a gene described in a report regarding a human immortalized monocytic cell was introduced thereinto. The immortalized monocytic cell line was cultured in an α-MEM medium containing 20% FBS, 50 ng / ml of M-CSF, and 50 ng / ml of GM-CSF, attained as a proliferable cell 4 to 5 weeks after the initiation of culturing, and then stored under liquid nitrogen using a commercially available cell preservation solution.

### (Confirmation of prepared cell)

In order to confirm that the prepared cell was a monocyte-like cell, a color evaluation by concentration, a measurement of a surface marker, a morphological observation by Giemsa staining, etc. were performed before the infection evaluation. The color evaluation by concentration was measured with a color of cells by visual observation when the cells were collected in a centrifuge tube. The measurement of a surface marker was made using a CD11 antibody and a CD14 antibody (both manufactured by Biolegend) with a flow cytometer CYFLOWSPACE (Sysmex). For staining, Giemsa staining or May-Giemsa staining was performed based on a known staining method using a commercially available staining solution (Sigma), and measurement was performed by a high-power microscope (400 to 1000 times).

### (Preparation of dendritic cell from immortalized monocytic cell)

The stored immortalized monocytic cell line was maintained and prepared by culturing in an α-MEM medium (containing 20% FBS, 50 ng / ml of M-CSF, and 50 ng / ml of GM-CSF) in an incubator at 37°C, 5% CO₂, and replacing the medium every 3 days. The dendritic cell was prepared by culturing the immortalized monocytic cell in the presence of 50 ng / ml of M-CSF, 50 ng / ml of GM-CSF, and 20 ng / ml of IL-4 for 3 days to thereby allow the cell to differentiate into a dendritic cell, and used for cell evaluation.

FIG. 1 illustrates confirmatory results of properties of the human monocyte-like cell prepared by the method in Example 1. These results indicate that the resultant human monocyte-like cell is a cell expressing surface markers specific for a monocyte-based cell (CD11 and CD14). Various expression amounts of CD14 are mixed, which suggests that not only a classical monocyte but also intermediate and non-classical monocyte-based cells are included.

FIG. 2 illustrates a Giemsa staining diagram of the human monocyte-like cell group of Example 1. It is seen from the Giemsa staining diagram that a portion of a profile of a cell membrane was protruded, which suggests that some of cells are in the process of being transformed into a dendritic-like cell due to physical stimulation.

### (Example 2) Production of human induced pluripotent stem cell-derived immortalized monocytic cell (iPS-ML)

A human induced pluripotent stem (iPS) cell-derived human immortalized monocytic cell was produced by reference to the previous reports (WO2012/043651 and Japanese Unexamined Patent Application, Publication No. 2017-131136). In detail, an undifferentiated iPS cell was seeded on a coating of a Matrigel as a basement membrane such as laminin 511, and culturing for inducing differentiation was initiated in an α-MEM medium (containing 20% FBS). Thereafter, the culturing was continued while the α-MEM medium (containing 20% FBS) was replaced every 3 days. Eighteen days after the initiation of inducing differentiation, the cell was dissociated by treating with a trypsin-EDTA (ethylenediamine tetraacetic acid)-collagenase liquid (37°C, 60 min), collected, and made into a cell suspension liquid through pipetting operation. Then, a cell derived from one dish having a diameter of 10 cm was suspended in 10 ml of a DMEM medium (containing 10% FBS), seeded on two dishes each having a diameter of 10 cm without a feeder cell and gelatin coating, and then left to stand. Three hours later, a cell which has not attached to the dish was collected and passed through a mesh of 100 micrometers (manufactured by BD Falcon, Cell Strainer) to thereby remove a cellular aggregate. Thus, a cell suspension liquid was obtained.

The cell suspension liquid obtained as described above was float-cultured in an α-MEM medium (containing 20% FBS, 50 ng / ml of M-CSF, and 100 ng / ml of GM-CSF). After about 3 to 9 days elapsed, it was observed that a floating or weakly-adherent cell appeared and gradually increased in number. This floating cell (iPS-MC) was collected and confirmed for expression of a leukocyte marker molecule CD45 and a myeloid-based cell marker CD11b or CD33 using a flow cytometer.

A gene reported as a mouse immortalized hematopoietic stem cell (Myc, Myb, Hob8, TLX1, E2A-pbx1, MLL, Ljx2, RARA, Hoxa9, Notch1, v-raf/v-myc, MYST3-NCOA2, Evil, HOXB6, HOXB4, β-catenin, Id1) or a gene described in a report regarding a human immortalized monocytic cell was introduced into the floating cell (iPS-MC) obtained as described above to thereby produce a human induced pluripotent stem cell-derived immortalized monocytic cell. This immortalized monocytic cell line was cultured in an α-MEM medium containing 20% FBS, 50 ng / ml of M-CSF, and 50 ng / ml of GM-CSF, obtained as a proliferable cell 4 to 5 weeks after the initiation of culturing, and then stored under liquid nitrogen using a commercially available cell preservation solution.

### (Example 3) Purification of prepared human immortalized monocyte-like cell through filter

The prepared human peripheral blood-derived immortalized monocytic cell (CD14-ML) and the human induced pluripotent stem cell-derived immortalized monocytic cell (iPS-ML) were evaluated for physical properties in supply of products. That is, when supplying the products, about ten-million to one hundred million cells are utilized at one time for evaluation in infection studies, and, therefore, a large amount of cells need to be cultured. In order to secure reproducibility of the evaluation, it is desired to minimize errors between lots and batches. For such control in amount, physical purification through a filter is envisaged to be superior to, for example, a flow cytometer from the viewpoints of cost and operating time. Therefore, the prepared human immortalized monocyte-like cell group was collected, and then, suspended in a culture solution and passed through a mesh filter (12 µm, manufactured by Murata Manufacturing Co., Ltd.) which had been wetted with the solution. A group which had been passed through was collected and centrifuged to thereby make the cells therein uniform in size.

FIG. 7 illustrates that measurement results with a flow cytometer of physical properties of the human immortalized monocyte-like cell group prepared according to the method of Examples 1 and 2 and purified through a filter. It is suggested that the human peripheral blood-derived monocytic cell (CD14-ML) produced in Example 1 is capable of being purified, for example, through a filter. Meanwhile, it is indicated that the human induced pluripotent stem (iPS) cell-derived immortalized monocytic cell (iPS-ML) is capable of being produced to be a certain size when measured after filtration through a filter, but a rate of dead cells is increased due to shredding of the cells. This suggests that the human peripheral blood-derived immortalized monocyte-like cell (CD14-ML) has a stronger cell membrane or stress-resistance than the human pluripotent stem cell-derived immortalized monocyte-like cell (iPS-ML).

### (Example 4) Transportation of prepared human monocyte-like cell

A human monocyte and dendritic-like cell group is desirably prepared and subjected to an infection evaluation at the same facility as each other, but may be needed to be transported. When the prepared cell group is transported to a facility for the infection evaluation, it was transported under the following conditions. About 1 x 10⁷ of each of the human immortalized monocytic cells or dendritic cells prepared according to the description in Examples 1 and 2 were transferred to a T-25 flask with a low adsorptive coating and the flask was filled with a medium, just before the transportation. The container was placed into a bio pouch with the container being sealed, further placed into a TACPACK (manufactured by Tamai Kasei Corporation), and then transported. The cells were transported at about 36°C using a temperature keeping agent in the box. Moreover, when a filter cap was used without sealing, an ANEROPACK (manufactured by Mitsubishi Gas Co., Ltd.) was placed together in the bio-pouch and transported while controlling O₂ and CO₂ concentrations. Transportation time was about 24 hours. After transportation (about 24 hours later), a cell viability was 70% or more and no change was observed in a cell state.

### (Example 5) Dengue virus infection test using human peripheral blood-derived immortalized monocytic cell (CD14-ML) and dendritic cell

After arriving at the transportation destination, the human peripheral blood-derived immortalized monocytic cell (CD14-ML) or the dendritic-like cell was immediately detached with trypsin and transferred from the T-25 flask to a centrifuge tube. The collected cell was used for evaluation. When the cell was not immediately used, the cell was transferred to, for example, a 100 mm dish and a medium was replaced every 3 days.

According to the plate design illustrated in FIG. 3, the human peripheral blood-derived immortalized monocytic cells (CD14-ML) (1 x 10⁷ cells) were seeded on a 96-well plate so that the final number of cells was 1 x 10⁵ / well. A well for the dendritic cell and a well for the monocytic cell were cultured in an incubator at 37°C, 5% CO₂ for 3 days in an α-MEM medium containing IL-4 (containing 20% FBS, 50 ng / ml of M-CSF, 50 ng / ml of GM-CSF, and 20 ng / ml of IL-4) and in an α-MEM medium without IL-4 (containing 20% FBS, 50 ng / ml of M-CSF, and 50 ng / ml of GM-CSF), respectively. The resultant cells were used for evaluation.

According to the plate design illustrated in FIG. 3, the prepared dengue virus of each serotype was mixed with the human peripheral blood-derived immortalized monocytic cell (CD14-ML) or the dendritic cell (1 x 10⁵ cells / well) in the 96-well plate to thereby infect the cell and cultured in an evaluation medium under conditions of 37°C, 5% CO₂. 72 hours later, a culture supernatant was collected and a progeny virus contained in the supernatant was measured for infection titer based on the plaque assay method. Specifically, a virus dilution liquid was inoculated into a Vero cell to thereby adsorb the virus thereto at 37°C for 1 hour, and then cultured in an MEM supplemented with 1% methyl cellulose at 37°C for 3 days. The cultured cell was fixed with acetone-methanol and then subjected to immunostaining. For the immunostaining, based on the known method, a mouse-derived flavivirus cross monoclonal antibody (D1-4G2-4-15 (ATCC (registered trademark) HB-112)) was used as a primary antibody (room temperature, 30 min) and was sequentially reacted with a biotin-labeled anti-mouse IgG antibody serving as a secondary antibody (room temperature, 30 min), an avidin-biotin complex (room temperature, 30 min), and a VIP substrate (room temperature, 10 min). The number of infected cells which were immunopositive was counted under a microscope and a virus titer was calculated.

Moreover, an infected human peripheral blood-derived immortalized monocytic cell (CD14-ML) after 72 hour culturing was fixed with acetone-methanol and subjected to immunostaining. For the immunostaining, based on the known method, a mouse-derived flavivirus cross monoclonal antibody (D1-4G2-4-15 (ATCC (registered trademark) HB-112)) was used as a primary antibody (room temperature, 30 min) and was sequentially reacted with a biotin-labeled anti-mouse IgG antibody serving as a secondary antibody (room temperature, 30 min), an avidin-biotin complex (room temperature, 30 min), and a VIP substrate (room temperature, 10 min). Infected cells which were immunopositive were observed and evaluated under a microscope.

The stained cells in each well are illustrated in FIG. 4. The cells infected with the virus were darkly stained and the human dendritic-like cells (Column 1 to 6: white arrow) were determined as being stained darker than a positive control (Column 7 to 12: black arrow) even when the virus was at a low concentration. Therefore, it was indicated that the dendritic cell induced from the monocyte having a proliferative capacity and produced by the method described in Example 1 was infected with the dengue virus. It is believed that an inhibitory capacity against dengue virus infection is capable of being evaluated by adding a pharmaceutical candidate substance under such experimental conditions. As illustrated in FIG. 5 (A), the monocyte-like cell (A1) was confirmed to be extremely strongly infected with the dengue virus type 1 compared with the control (A7) which was sensitized to the virus at the same concentration. As a result, the cell was revealed to be more suitable for quantitatively evaluating a drug.

FIG. 6 illustrates a virus release amount due to sensitization to the dengue virus. Since the virus release amount from the dendritic cell is larger than from the positive control K562 cell, the dendritic cell was revealed to be more suitable for proliferating a virus and obtaining a mutant thereof using a cell more closely related to those of human.

### (Example 6) Dengue virus infection test using human monocyte-like and dendritic-like cell 2

After the human peripheral blood-derived immortalized monocytic cell (CD14-ML) and the human induced pluripotent stem cell-derived immortalized monocytic cell (iPS-ML) arrived at the transportation destination, the cells were immediately detached with, for example, trypsin and transferred from the T-25 flask to a centrifuge tube. After the cell was collected, the cell was transferred to, for example, a 100 mm dish and a medium was replaced every 3 days.

According to the plate design illustrated in FIG. 8, the human monocyte-like cells (1 x 10⁷ cells) were seeded on a 96-well plate so that the final number of cells was 1 x 10⁵ / well. When the monocyte-like cell was evaluated as is, the cell was cultured in an α-MEM medium (20% FBS, 50 ng / ml of M-CSF, 50 ng / ml of GM-CSF) and when the dendritic-like cell was evaluated, the cell was cultured in an IL-4 containing α-MEM medium (containing 20% FBS, 50 ng / ml of M-CSF, 50 ng / ml of GM-CSF, and 20 ng / ml of IL-4), both were performed in an incubator at 37°C, 5% CO₂ for 3 days. Three days later, the resultant cells were used for evaluation.

According to the plate design illustrated in FIG. 8, the dengue virus of each serotype which had been adjusted for an MOI concentration was mixed with the human dendritic-like cell (1 x 10⁵ cells / well) in the 96-well plate to thereby infect the cell and cultured in an evaluation medium under conditions of 37°C, 5% CO₂. 72 hours later, a culture supernatant was collected and a progeny virus contained in the supernatant was measured for infection titer based on a plaque assay method. Specifically, based on the plate design illustrated in FIG. 9, a supernatant in each well in FIG. 8 (into which the proliferated virus was thought to be released) was inoculated into a Vero cell according to a dilution rate to thereby adsorb the virus thereto at 37°C for 1 hour, and then cultured in an MEM supplemented with 1% methyl cellulose at 37°C for 3 days. The cultured cell was fixed with acetone-methanol and then subjected to immunostaining. For the immunostaining, based on the known method, a mouse-derived flavivirus cross monoclonal antibody (D1-4G2-4-15 (ATCC (registered trademark) HB-112)) was used as a primary antibody (room temperature, 30 min) and was sequentially reacted with a biotin-labeled anti-mouse IgG antibody serving as a secondary antibody (room temperature, 30 min), an avidin-biotin complex (room temperature, 30 min), and a VIP substrate (room temperature, 10 min).

FIG. 10 illustrates results of the plaque assay of the human peripheral blood-derived dendritic-like cell(CD14-DC) and the human induced pluripotent stem (iPS) cell-derived dendritic-like cell(iPS-DC) using the dengue virus type 4 (DENV-4). When the dengue virus invades the human dendritic-like cell, proliferates therein, and releases a progeny virus, the collected supernatant contains the released progeny virus. Therefore, it is stained after secondary culturing on the Vero cell. Compared with a detection limit of the virus in a commonly used K562 cell or Vero cell, the virus was capable of proliferating and being detected even at extremely low concentrations (concentrations indicated by open and black arrows; sensitivity is 100 times or more, in some viruses, 10000 times or more of those in the existing cells) in the human peripheral blood-derived dendritic-like cell (CD14-DC) and the human induced pluripotent stem (iPS) cell-derived dendritic-like cell (iPS-DC). It was revealed that evaluation under such experimental conditions makes it possible to test or isolate viruses with high sensitivity and, in addition, to evaluate drugs since an environment under which viruses which was difficult to culture due to its low concentration including other flaviviruses can be cultured is provided.

FIG. 11 illustrates results of the plaque assay of the human peripheral blood-derived dendritic-like cell (CD14-DC) using the dengue virus type 1 (DENV-1). Formation of the plaque reflects a state of the virus. In the present test, small spots (white arrow) and large spots (black arrow) were observed. This result suggests change in the state of the virus and a possibility of occurring a viral mutation even after only one infection experiment. It was revealed that a novel research and development method (method for establishing, developing, and producing vaccine strains) may be provided for the existing problem that it takes a long time for establishment, development, and production of vaccine candidate strains due to difficulty of causing mutations.

## Claims

1. A method for maintenance culturing a monocyte- or dendritic cell-mediated infectious microorganism, the method comprising:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism; and
culturing the infected cell.

2. A method for maintenance culturing a monocyte- or dendritic cell-mediated infectious microorganism, the method comprising:
introducing a cMYC gene, and at least one gene selected from a BMI1 gene, an EZH2 gene, an MDM2 gene, an MDM4 gene, an HIF1A gene, a BCL2 gene, and an LYL1 gene into a monocyte;
optionally inducing the monocyte to differentiate into a dendritic cell;
infecting the resultant monocyte or dendritic cell with the infectious microorganism; and
culturing the infected cell.

3. A method for identifying a therapeutic agent against an infection with a monocyte- or dendritic cell-mediated infectious microorganism, the method comprising:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism in the presence of a test drug;
measuring an infection level with the infectious microorganism in the monocyte or the dendritic cell; and
determining whether the test drug has a therapeutic effect against the infection based on the measured infection with the infectious microorganism,
wherein the test drug is determined to have the therapeutic effect against the infection if a level of the measured infection with the infectious microorganism is lower than a control infection level which is a level when infecting the cell with the infectious microorganism in the absence of the test drug.

4. A method for producing a mutant of a monocyte- or dendritic cell-mediated infectious microorganism, the method comprising:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism;
culturing the infected monocyte or dendritic cell to thereby proliferate the infectious microorganism; and
isolating a mutant from the proliferated infectious microorganism.

5. A method for producing a vaccine against a monocyte- or dendritic cell-mediated infectious microorganism, the method comprising:
infecting a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte with the infectious microorganism;
culturing the infected monocyte or dendritic cell to thereby proliferate the infectious microorganism; and
collecting the proliferated infectious microorganism.

6. The method according to claim 6, further comprising inactivating the collected infectious microorganism.

7. A method for separating a monocyte- or dendritic cell-mediated infectious microorganism from blood derived from a patient infected with the infectious microorganism, the method comprising:
bringing a monocyte to which a cMYC gene, and at least one gene selected from a BMI1 gene, an EZH2 gene, an MDM2 gene, an MDM4 gene, an HIF1A gene, a BCL2 gene, and an LYL1 gene have been introduced, or a dendritic cell which has been induced to be differentiated from the monocyte into contact with the blood derived from a patient;
culturing the infected cell; and
detecting and/or identifying a microorganism in the cultured cell to thereby specify the infectious microorganism.

8. The method according to any one of claims 1 to 7, wherein the monocyte is a cell obtained by inducing a human pluripotent stem cell or a human hematopoietic stem cell to differentiate, or a cell obtained by isolating from human peripheral blood.

9. The method according to claim 8, wherein the human pluripotent stem cell is an iPS cell.

10. The method according to any one of claims 1 to 7, further comprising preparing the monocyte by inducing a human pluripotent stem cell or a human hematopoietic stem cell to differentiate, or preparing the monocyte by isolating from human peripheral blood.

11. The method according to claim 10, wherein the human pluripotent stem cell is an iPS cell.

12. The method according to any one of claims 1 to 11, wherein the infectious microorganism is a dengue virus.

13. The method according to any one of claims 1 to 12, wherein a culturing time is 24 to 96 hours.

14. The method according to claim 4, wherein a number of times of culturing is 1 to 5.

15. The method according to any one of claims 1 to 14, wherein the least one gene selected from a BMI1 gene, an EZH2 gene, an MDM2 gene, an MDM4 gene, an HIF1A gene, a BCL2 gene, and an LYL1 gene is a BMI1 gene and a BCL2 gene or an LYL1 gene.
